# FASCICULE DE BREVET EUROPEEN

(11) **EP 0 615 454 B1**
(45) Date de publication et mention de la délivrance du brevet: **17.01.2001**
(21) Numéro de dépôt: 93921970.5
(22) Date de dépôt: 30.09.1993
(51) Int. Cl.: A61L 24/00

(54) **PROCEDE DE PREPARATION D'UNE COLLE BIOLOGIQUE ENRICHIE EN FACTEURS PLAQUETTAIRES ET APPLICATION**
VERFAHREN ZUR HERSTELLUNG EINES MIT PLÄTTCHENFAKTOREN ANGEREICHERTEN BIOLOGISCHEN KLEBSTOFFES UND SEINE VERWENDUNG
METHOD OF PREPARING A BIOLOGICAL ADHESIVE ENRICHED WITH PLATELET FACTORS, AND APPLICATION

(30) Priorité: 30.09.1992 FR 9211643
(43) Date de publication de la demande: 21.09.1994
(73) Titulaire: INOTEB, F-56920 Saint-Gonnery (FR)
(72) Inventeur: PATAT, Jean-Louis, F-75017 Paris (FR); DELMAS, Olivier, F-37250 Montbazon (FR); SCHMITTHAEUSLER, Roland, F-78180 Montigny-le-Bretonneux (FR)
(74) Mandataire: Hubert, Philippe
(86) Numéro de dépôt international: FR9300954
(87) Numéro de publication internationale: WO9407548

(56) Documents cités:
- EP-A- 0 068 048
- EP-A- 0 305 243
- WO-A-86/03122
- WO-A-91/09573
- WO-A-92/09301
- GB-A- 2 041 942

## Description

L'invention a pour objet l'obtention d'une colle biologique contenant des protéines plasmatiques humaines coagulables. La colle biologique obtenue selon l'invention contient notamment des facteurs de croissance d'origine plaquettaire.

On sait que des concentrés de protéines coagulables par la thrombine, ou des solutions de fibrinogène, sont utilisés dans la réalisation d'un matériau collant permettant notamment de réunir les tissus vivants tout en exerçant une action hémostatique ; voir par exemple le brevet FR-2.448.900. Ce matériau collant est couramment appelé "colle biologique" et est utilisé en chirurgie.

Les colles biologiques permettent de reproduire, dans des conditions physiologiques, la phase finale du processus de la coagulation. Pour cela, on mélange extemporanément, sur le tissu vivant à traiter, une solution contenant du fibrinogène et du facteur XIII avec une solution contenant de la thrombine et des ions calcium. Le fibrinogène, en présence de thrombine et d'ions calcium, est transformé en fibrine. Les monomères de fibrine s'assemblent pour former des polymères de fibrine et ces polymères sont réticulés par l'action du facteur XIII activé. Le facteur XIII, activé sous l'action de la thrombine en présence d'ions calcium, est une transamidase qui établit des liaisons peptidiques entre les chaînes de fibrine avec formation d'un réseau.

Dans la présente demande, on appellera "colle biologique" un concentré de protéines coagulables par la thrombine, bien qu'en fait ce soit seulement en présence de thrombine et de sels de calcium (ou après activation de la prothrombine qu'il contient) qu'un tel concentré constitue véritablement une colle.

La formation du réseau de fibrine est responsable de l'activité hémostatique de la colle biologique. Par ailleurs, la fibrine adhère aux tissus environnants, notamment par l'intermédiaire de la fibronectine et du collagène. En outre, le facteur XIII activé établit des liaisons peptidiques entre la fibrine et la fibronectine, ce qui renforce les propriétés adhésives.

On sait que le caillot de fibrine disparaît progressivement, in vivo, sous l'action d'une enzyme protéolytique appelée plasmine, ce qui limite la durée du collage. Il est toutefois possible de renforcer la durée d'action des colles biologiques en leur ajoutant par exemple de l'alpha 2-antiplasmine ou un inhibiteur des protéases tel que l'aprotinine, ou encore de l'acide epsilon-aminocaproïque.

Les applications des colles biologiques sont nombreuses, en particulier en chirurgie pour éviter des saignements, pour remplacer les fils de sutures ou pour renforcer celles-ci.

Cependant, les colles biologiques n'ont pas d'activité particulière propre à favoriser la cicatrisation.

On sait que la cicatrisation est favorisée notamment par certains facteurs de croissance agissant directement sur des cellules-cibles présentes au niveau d'une plaie. Ces facteurs de croissance induisent la multiplication ou la différenciation, ou encore l'attraction cellulaire (chimiotactisme). Les plaquettes sanguines (ou thrombocytes) sont une des sources principales de facteurs de croissance dans le sang.

Lors d'une lésion cellulaire in vivo, les thrombocytes libèrent les facteurs de croissance contenus dans des granules, sous l'action d'activateurs des thrombocytes, dont la thrombine. Les surnageants de plaquettes activées renferment une grande diversité de molécules, comme par exemple le facteur de croissance dérivé des plaquettes, ou PDGF (Platelet-derived growth factor), le "Transforming growth factor bêta",le "Basic fibroblast growth factor", le "Platelet factor 4", le "Platelet-derived endothelial growth factor", l'"Heparin-binding epidermal growth factor", le "Insulin-like growth factor 1", le "Connective-tissue activating peptide III", la bêta-thromboglobuline, l'"Epidermal growth factor", le plasminogène, le facteur Von Willebrand, le fibrinogène, la sérotonine, l'histamine, l'adénosine di-et triphosphate, la fibronectine, la vitronectine, le facteur XIII plaquettaire, des enzymes protéolytiques ou glycolytiques, les métabolites de l'acide arachidonique etc.... ; voir notamment H.L. Wong & S.M. Wahl, In "Peptide Growth Factors and their Receptors" Sporn & Roberts Eds., Springer-Verlag. Berlin, p.510 ; R.A. Terkeltaub & M.H. Ginsberg, In "The Molecular and Cellular Biology of Wound repair", Clark & Henson Eds, Plenum Press, New York, p.38.

Les extraits plaquettaires renferment une activité mitogène élevée, et leur effet cicatrisant est connu ; voir notamment D.Knighton et al., Ann. Surg., 196, 379-388 (1982) ; D.M. Carter et al., In "Growth factors and other aspects in Wound Healing", Barbul. Pines, Cadwell Hunt Eds, Alan R.Liss Inc., New York 1988, p.303-317 ; brevet US 4,760,131 et demandes de brevet PCT WO 86/03122, WO 88/03409 et WO 89/05656.

On a maintenant découvert qu'il est possible d'obtenir une colle biologique améliorée, favorisant notamment la cicatrisation, grâce à un procédé permettant de réunir à la fois les protéines coagulables par la thrombine et des facteurs plaquettaires.

Les modes de préparation des concentrés de protéines coagulables par la thrombine sont connus. La matière première est constituée de plasma sanguin, c'est-à-dire de sang dont on a éliminé les cellules sanguines. Autrement dit, il s'agit d'un produit appauvri en plaquettes. Le procédé consiste essentiellement à précipiter le fibrinogène soit par l'éthanol, selon la méthode de Cohn, soit par l'obtention d'un cryoprécipité. On sait que la préparation d'un cryoprécipité consiste à congeler un plasma puis à le décongeler à une température supérieure à 0 et inférieure à 6°C, généralement comprise entre +1 et +4°C. La fraction solide qui demeure, et qui contient notamment le fibrinogène et la fibronectine, est appelée cryoprécipité et peut être séparée de la fraction liquide par centrifugation. Le volume du cryoprécipité est généralement compris entre 1/25 et 1/100 du volume du plasma de départ. Les protéines plasmatiques insolubles dans les conditions d'obtention du cryoprécipité sont donc concentrées d'un facteur 25 à 100 au cours de la cryoprécipitation. Mais les préparations ainsi obtenues sont pauvres en facteurs de croissance. En particulier les colles biologiques préparées selon des procédés industriels connus ne renferment pas d'activité mitogène notable.

On a maintenant découvert qu'en utilisant comme produit de départ un plasma enrichi en plaquettes, de nombreux facteurs plaquettaires, bien que normalement solubles dans les conditions d'obtention du cryoprécipité, se trouvent retenus dans ledit cryoprécipité en proportions très importantes, alors qu'on devait normalement s'attendre à une répartition uniforme de ces facteurs entre la fraction liquide et le cryoprécipité obtenus.

Le procédé de l'invention permet d'obtenir un produit combinant les propriétés hémostatiques et adhésives des concentrés de protéines coagulables par la thrombine et les activités cicatrisantes des extraits plaquettaires, sans qu'il soit nécessaire de procéder à deux préparations distinctes.

C'est ainsi que grâce au procédé de l'invention, le facteur de concentration du PDGF, dans le cryoprécipité, est généralement supérieur à 10. Le rendement en PDGF est généralement supérieur à 20 ng par milliard de plaquettes présentes dans le plasma de départ.

L'invention a donc pour objet un procédé de préparation d'une colle biologique contenant un concentré de protéines coagulables par la thrombine, caractérisée par le fait qu'elle contient au moins un facteur de croissance d'origine plaquettaire.

Ledit facteur de croissance est notamment le PDGF.

La colle biologique obtenue selon l'invention contient généralement au moins 20 ng/ml, et le plus souvent au moins 50 ng/ml de PDGF. Généralement, elle peut contenir de 20 à 600 ng/ml de PDGF.

La colle biologique contient d'autres produits présents normalement dans les extraits plaquettaires, et en particulier la bêta-thromboglobuline, l'inhibiteur de type 1 de l'activateur du plasminogène (PAI-1) et le facteur XIII plaquettaire (Facteur XIII-A). Elle contient par exemple au moins 10 µg/ml, et en particulier de 10 à 300 µg/ml, de bêta-thromboglobuline, au moins 1 µg/ml de PAI-1 et en particulier de 5 à 40 µg/ml, de PAI-1. La proportion de facteur XIII-A par rapport au Facteur XIII-S est au moins 1,2 fois plus élevée que la proportion de ces facteurs dans un plasma normal, et en particulier de 1,2 à 4 fois plus élevée.

La colle biologique de l'invention contient par exemple :
- protéines : 60 - 200 g/l,
- fibrinogène : 30 - 150 g/l, en particulier 50-100 g/l,
- fibronectine : 6 - 14 g/l, notamment 8 - 12 g/1,
- facteur XIII : 10 - 60 unités équivalent plasma,
- albumine : 10 - 38 g/l, en particulier 18-30 g/l.

L'unité équivalent plasma correspond à la concentration plasmatique normale en facteur XIII présente dans un pool de plasmas.

La concentration en protéines est déterminée par la méthode du Biuret. La concentration en fibrinogène est déterminée par la méthode pondérale, la concentration en fibronectine et en PAI-1 par un test ELISA (Stago), selon les instructions du fabricant, l'activité en facteur XIII par un test de libération d'amoniac provoquée par l'incorportion d'éthylester de glycine sur un substrat peptidique (Réactifs Berichrom F XIII, Behring), selon les recommandations du fabricant, le facteur XIII-A et XIII-S par la méthode de Laurell en utilisant des antisera spéfifiques (Stago). La concentration en albumine est déterminée par électrophorèse sur Acétate de Cellulose (Sebia), selon les recommandations du fabricant. La teneur en protéine de la bande d'électrophorèse caractéristique de l'albumine est déterminée par densitométrie.

La colle biologique contient les facteurs de la coagulation en quantité suffisante pour lui conférer la propriété de coaguler en activant soit la voie intrinsèque de la coagulation, soit la voie extrinsèque de la coagulation. La colle biologique contient par exemple entre 0,5 et 1,5 fois la concentration plasmatique normale en prothrombine, déterminée soit par la méthode de Laurell en utilisant un antiserum spécifique (Stago), soit par la méthode chronométrique (Stago déficient II, Stago), selon les recommandations du fabricant.

Une dilution au 1/2 dans un tampon physiologique de la colle biologique présente un temps de Quick équivalent à celui d'un plasma normal dilué entre 1/2 et 1/6. Le temps de Quick explore la capacité à activer la voie extrinsèque de la coagulation. Il est déterminé à l'aide du réactif de marque "Neoplastine" (Stago), contenant de la thromboplastine, selon les recommandations du fabricant.

Une dilution au 1/2 dans un tampon physiologique de la colle biologique présente un temps de Kaolin équivalent à celui d'un plasma normal dilué entre 1:1 et 1:4. Le temps de Kaolin explore la capacité d'activation de la voie intrinsèque de la coagulation. Il est déterminé à l'aide des réactifs C.K. PREST (Stago), selon les recommandations du fabricant, sauf que la céphaline est omise lors de la réalisation du test.

Le procédé de préparation de la colle biologique selon l'invention contient des facteurs plaquettaires en quantité suffisante pour lui conférer des propriétés mitogènes, pouvant être mise en évidence selon les méthodes classiques.

L'activité mitogène de la colle biologique selon l'invention est par exemple telle que diluée au 1/4000e (et même, généralement, au 1/5000e), elle possède une activité mitogène au moins égale à celle d'une solution contenant 0,1 ng/ml de PDGF dans un test de mesure de l'incorporation de thymidine tritiée par des cellules 3T3 stimulées à une phase d'arrêt de croissance.

Un tel test est décrit par exemple par HART C.E. et al., Biochemistry vol.29, 166-172 (1990).

La cellule 3T3 est une cellule d'embryon de souris Swiss (lignée ATCC CCL 92). Une lignée provenant d'un sous-clonage est également disponible (NIH/3T3 ; ATCC CRL 1658).

La colle biologique peut être constituée essentiellement d'un cryoprécipité issu d'un plasma enrichi en plaquettes, et l'invention concerne un procédé de préparation d'une colle biologique dans lequel on prépare et recueille un cryoprécipité à partir d'un plasma congelé, caractérisé par le fait que l'on utilise comme plasma de départ un plasma enrichi en plaquettes.

Autrement dit, le procédé de l'invention est caractérisé par le fait qu'avant de préparer le cryoprécipité, on prépare un plasma enrichi en plaquettes. L'opération d'enrichissement du plasma en plaquettes est effectuée selon les méthodes usuelles, notamment par centrifugation du sang prélevé.

Le plasma enrichi en plaquettes contient généralement au moins 50 millions de plaquettes par ml, notamment de 100 millions à 1 milliard de plaquettes environ par ml, et par exemple de 100 millions à 500 millions environ par ml.

Les conditions d'obtention du cryoprécipité sont les conditions classiques. Par exemple on congèle à une température non supérieure à -15°C, par exemple comprise entre -15 et -60°C. On maintient le produit à cette température pendant au moins un temps suffisant pour permettre un équilibre thermique dans la masse du produit. Ce temps est par exemple de l'ordre de 12 heures à 72 heures.

Le plasma est ensuite décongelé à une température supérieure à 0°C mais inférieure à la température à laquelle l'ensemble du produit congelé se liquéfie. On opère généralement entre +1 et +6°C, et en particulier vers +4°C. La durée d'incubation à la température de décongélation est suffisante pour permettre de réaliser un équilibre thermique ; cette durée est comprise par exemple entre 12 et 24 heures.

On peut ensuite séparer le cryoprécipité de la fraction liquide. Pour cela, on centrifuge le plasma décongelé à une température suffisamment élevée pour ne pas le recongeler, et suffisamment basse pour éviter la liquéfaction du cryoprécipité, puis on élimine le surnageant selon les méthodes connues, et l'on recueille le cryoprécipité qui constitue le culot de centrifugation. Le volume du cryoprécipité représente généralement le 1/25e à 1/100e du volume de plasma initial.

Généralement la centrifugation est effectuée à une température comprise entre +1 et +6°C.

Dans le cas où l'on ne souhaite pas utiliser immédiatement la colle biologique constituée par le cryoprécipité obtenu, on peut la recongeler, par exemple à -20°C.

Lorsque la colle biologique est destinée à être utilisée immédiatement, il convient de la liquéfier par chauffage à une température suffisante, habituellement comprise entre 32 et 38°C.

La simplicité du procédé d'obtention de la colle biologique enrichie en facteurs plaquettaires selon l'invention permet d'appliquer ce procédé à la réalisation d'une colle biologique autologue, c'est-à-dire obtenue à partir du plasma provenant d'un seul donneur, en vue de l'utilisation chez ce donneur. L'un des avantages de ce procédé est que toutes les opérations, y compris l'enrichissement du plasma en plaquettes, peuvent être effectuées dans le système de poches utilisé pour le prélèvement du sang d'un donnneur.

On peut bien entendu préparer également la colle biologique à partir de plasma provenant de plusieurs donneurs identifiés.

Le produit de départ du procédé de l'invention est un plasma enrichi en plaquettes. La préparation d'un tel plasma enrichi en plaquettes est connue en soi. Elle peut être réalisée par exemple selon la méthode suivante. Du sang total, recueilli lors d'un don de sang, dans un système à poche triple (Maco-pharma par exemple), est centrifugé dans une centrifugeuse Jouan K110 à 3000xg pendant 4 minutes. Le plasma surnageant, riche en plaquettes, est transvasé dans la poche de transfert 3 du système de triple poche, à l'aide d'un extracteur de plasma. La poche 3 est séparée du reste du système par soudure de la tubulure. C'est cette poche qui sera utilisée pour la fabrication selon l'invention du concentré de protéines coagulables par la thrombine.

L'invention a également pour objet l'utilisation autre que dans une méthode de traitement thérapeutique ou chirurgical du corps humain ou animal de la colle biologique pouvant être obtenue selon le procédé mentionné ci-dessus.

Cette utilisation peut être effectuée selon les méthodes connues en soi. Le principe de cette utilisation consiste à provoquer la formation de fibrine par transformation du fibrinogène. Cette transformation est réalisée par l'action de la thrombine. La thrombine peut être de la thrombine exogène ajoutée, selon les méthodes connues en soi : pour cela, on mélange la colle biologique obtenue telle que décrite précédemment avec une solution aqueuse contenant de la thrombine et des ions calcium afin de déclencher la réaction de coagulation par transformation du fibrinogène en fibrine.

On peut utiliser notamment une solution aqueuse contenant de 0,5 à 500 U NIH/ml de thrombine et une concentration en ions calcium (notamment sous le forme de chlorure de calcium) comprise par exemple entre 5 et 100 mM, en particulier entre 20 et 60 mM.

Le mélange avec la solution de thrombine est effectué de préférence in situ, sur les tissus à traiter.

Généralement, on mélange le cryoprécipité liquéfié et la solution aqueuse de thrombine dans des proportions volumiques de 5:1 à 1:2.

Si désiré, on ajoute à la colle finale, ou à l'un de ses constituants avant leur mélange, un inhibiteur de plasmine ou analogue (alpha 2-antiplasmine, aprotinine, acide epsilon-aminocaproïque, acide tranexamique par exemple).

On peut aussi tirer avantageusement parti de la présence de prothrombine et d'autres facteurs de coagulation dans la colle biologique de l'invention, et activer soit la voie extrinsèque, soit la voie intrinsèque de la coagulation.

L'invention a donc notamment pour objet l'utilisation de la colle biologique obtenue comme indiqué précédemment, cette utilisation étant caractérisée par le fait que l'on ajoute à ladite colle biologique au moins un agent favorisant l'activation de la prothrombine endogène. On déclenche aussi le phénomène de coagulation, en opérant de préférence à température voisine de 37°C.

Pour cela, il suffit d'ajouter à la colle biologique une solution aqueuse contenant des ions calcium (par exemple 5-100 mM, en particulier 20 - 60 mM).

Si on veut accélérer cette coagulation, on peut opérer comme indiqué ci-après, à l'aide d'activateurs.

Pour activer la voie intrinsèque, on peut mélanger la colle biologique avec une solution aqueuse contenant des ions calcium (par exemple 5 - 100 mM, en particulier 20 - 60 mM), et la mettre en contact avec au moins un activateur connu du Facteur XII de la coagulation, et de manière plus générale avec des surfaces ou des composés solides, par exemple sous forme de poudre, insolubles dans la colle biologique et présentant des charges négatives, comme des silicates, (notamment le Kaolin), la silice, le verre de silice, des cristaux de carbonate de calcium, comme par exemple l'aragonite, provenant par exemple de squelettes de corail, des cristaux de tri-calcium dicitrate. Au bout d'un temps suffisant, qui peut être déterminé par de simples expériences de routine, on sépare la colle biologique desdits composés insolubles par filtration. On peut opérer par exemple dans une colonne munie d'un filtre.

Pour activer la voie extrinsèque de la coagulation, on peut mélanger la colle biologique avec une solution aqueuse contenant des ions calcium 5 - 100 mM (en particulier 20 - 60 mM) et de la thromboplastine tissulaire, par exemple d'origine animale, ou de la thromboplastine produite par génie génétique.

L'un des avantages importants de la colle biologique obtenue selon l'invention est donc qu'elle contient de la prothrombine en quantité suffisante pour permettre de déclencher la coagulation de la colle sans addition de thrombine exogène, ce qui réduit les risques de contamination accidentelle. Le procédé de l'invention est donc particulièrement adapté aux besoins actuels d'utilisation de produits autologues.

La colle biologique peut être utilisée notamment dans le traitement des plaies traumatiques ou chirurgicales, dans la mise en place et le maintien des greffes (notamment greffes de peau, d'os, etc...). Elle peut également être utilisée pour fixer des pièces de prothèse, y compris de prothèses osseuses, de pièces d'ostéosynthèse et de prothèses dentaires, et aussi pour fixer et maintenir assemblés des matériaux de comblement osseux, notamment des matériaux de comblement résorbables à base de carbonate de calcium (par exemple à base de squelette de corail ou de coquille de Pinctada margaritifera), d'hydroxyapatite ou de polymères biodégradables comme le poly(acide lactique).

### EXEMPLE 1

On a préparé un plasma humain enrichi en plaquettes, comme décrit précédemment.

Le plasma enrichi ainsi obtenu contient 154 millions de plaquettes par ml.

La poche de transfert renfermant 230 ml dudit plasma enrichi en plaquettes est placée dans une chambre froide à -40°C pendant 72 heures. La poche de plasma est ensuite placée dans un réfrigérateur à une température de 4°C pendant 20 heures.La poche de plasma est ensuite centrifugée pendant 20 minutes à 3.000 tours par minute, toujours à la température de +4°C. Après centrifugation, la poche est posée horizontalement, une tubulure de prélèvement est mise en place et le surnageant liquide est soutiré par aspiration.

Le résidu solide contenu dans la poche constitue le cryoprécipité qui peut être conservé par recongélation à une température de -20°C jusqu'à son utilisation.

Lorsqu'on souhaite utiliser immédiatement le cryoprécipité, on place la poche au bain-marie, par exemple à une température de 37°C. Après 15 minutes d'incubation la solution résiduelle est prélevée. Son volume est de 3 ml environ.

Analyse du produit obtenu : voir tableau 1 ci-après.

### EXEMPLE 2

On opère de façon analogue à celle décrite à l'exemple 1. Le volume de plasma traité est de 250 ml. Le plasma renfermait au départ, 256 millions de plaquettes par ml. Le volume de la solution finale récupérée, provenant de la liquéfaction du cryoprécipité, est de 5 ml.

Analyse : voir tableau 1.

La teneur en PDGF du surnageant obtenu après centrifugation du plasma décongelé n'est que de 5,9 ng/ml.

On voit (par comparaison avec le Tableau I) que la quasi-totalité du PDGF se retrouve dans le cryoprécipité.

**TABLEAU I**

| | Exemple 1 | Exemple 2 |
|---|---|---|
| Protéines(1), (mg/ml) | 61 | 89,6 |
| PDGF(2), (ng/ml) | 282 | 310 |
| Bêta-Thromboglobuline(3) (µg/ml) | 127 | 130 |
| Coagulable par la thrombine(4) | OUI | OUI |
| Activité mitogène(5) (activité spécifique) | 104 | non-mesuré |

| | | |
|---|---|---|
| (1) Déterminé par la méthode de Bradford (Réactif Biorad référence 500-0006) | | |
| (2) PDGF : facteur de croissance dérivé de plaquettes (Platelet Derived Growth Factor), mesuré par un test "ELISA" | | |
| (3) Mesuré par un test "ELISA" (Stago, France, référence 0419) | | |
| (4) Déterminé en mélangeant un volume de cryoprécipité liquéfié à un volume d'une solution de thrombine à 500 U.N.I.H. et de chlorure de calcium 50 mM. Après 1 minute, on observe la formation d'un caillot. | | |
| (5) L'activité spécifique est déterminée selon le protocole décrit ci-après. La radioactivité obtenue avec le témoin est de 2.000 cpm. A 4.000 cpm, la concentration en protéines est de 0,0096 mg/ml dans l'exemple 1. A titre indicatif, l'activité spécifique du Tissucol (marque de commerce désignant une colle biologique vendue par IMMUNO) mesurée par la même méthode, est de 0,22. | | |

Le protocole mesure de l'activité mitogène était le suivant:

Des cellules de fibroblastes sont isolées à partir du prépuce d'un enfant âgé de 6 mois.

Les cellules sont mises en suspension dans du milieu DMEM (Gibco) renfermant 10% de sérum de veau fétal. On ensemence chaque puits d'une plaque de microtitration à 96 puits avec 200 µl de la suspension à 40.000 cellules/ml. Après 3 jours d'incubation à 37°C, en athmosphère à 5% de CO₂, le milieu de culture est remplacé par un milieu DMEM (Gibco) à 8% de sérum de veau nouveau-né.

Après 3 jours d'incubation supplémentaires, 50 p1 d'une solution de protéines coagulables par la thrombine (échantillon) sont ajoutés. Chaque échantillon est testé à différentes dilutions dans du DMEM, et en triple. 50 µl de DMEM sont inoculés dans des puits témoin. La microplaque est incubée dans les mêmes conditions pendant 24 heures. Six heures avant la fin de l'incubation, 50 µl d'une solution de thymidine tritiée à 10 µCi/ml sont ajoutés dans chaque puits. Après l'incubation, les puits sont lavés par une solution physiologique. Les cellules sont alors décollées du puits par une solution de trypsine à 0,25%, en présence d'éthylène-diamine-tétra-acétate (solution Gibco). Les cellules sont alors récoltées sur un filtre, grâce à un collecteur de cellules de type "Skatron". Les filtres sont alors séchés et la radioactivité est mesurée en présence d'un liquide scintillant.

Détermination de l'activité spécifique :

On reporte sur un graphe, en abcisse, la concentration en protéines de l'échantillon dans le puits (exprimée en mg/ml) et en ordonnée la radioactivité correspondante. On détermine graphiquement, par interpolation, la concentration en protéines de l'échantillon donnant un taux de radioactivité double de celle des puits témoin. L'inverse de cette concentration est appelée l'activité spécifique de l'échantillon.

### EXEMPLE 3 :

On opère de façon analogue à celle de l'exemple 1. Les résultats des analyses (moyennes de plusieurs expériences) sont consignés dans le tableau II

**TABLEAU II**

| | Nb d'exp | Moyenne | Val. mini | Val. maxi |
|---|---|---|---|---|
| Concentration en plaquettes dans le plasma de départ 10⁶/ml | 15 | 350 | 221 | 552 |
| Protéines g/l | 15 | 120 | 95 | 160 |
| Fibrinogène g/l | 15 | 69 | 52 | 93 |
| Fibronectine g/l | 9 | 7,8 | 6 | 13 |
| Facteur XIII UEP * | 15 | 28 | 15 | 57 |
| F XIII-A/F XIII-S | 4 | 2,9 | 2,14 | 3,7 |
| PDGF ng/ml | 4 | 400 | 300 | 600 |
| bêta-thromboglobuline µg/ml | 9 | 200 | 184 | 250 |
| Albumine g/l | 6 | 25,1 | 19,8 | 35,9 |
| PAI-1 µg/ml | 5 | 19 | 14 | 24 |

| | | | | |
|---|---|---|---|---|
| * UEP : Unité équivalent plasma | | | | |

### EXEMPLE 4

On prépare une colle biologique de façon analogue à celle décrite dans l'exemple 1. Dans un tube, on mélange successivement 150 µl de colle biologique, 150 µl de serum physiologique, 300 µl d'une solution aqueuse de chlorure de calcium à 25 mM et, soit 300 µl d'une suspension de Kaolin,soit 300 µl d'une suspension de poudre de corail, soit 300 µl de sérum physiologique. Le tube est incubé à 37°C au bain-marie. On suit l'apparition d'un coagulum dans le tube.

En présence de Kaolin, le coagulum se forme après 4 min d'incubation. En présence de poudre de corail au lieu de Kaolin, le coagulum se forme 7 min. La préparation sans Kaolin ni corail (obtenue par simple addition de sérum physiologique) coagule au bout de 9 min.

## Revendications

1. Procédé de préparation d'une colle biologique dans lequel on prépare et recueille un cryoprécipité, constituant ladite colle, à partir d'un plasma congelé, caractérisé par le fait que l'on utilise comme plasma de départ un plasma enrichi en plaquettes.

2. Procédé selon la revendication 1, caractérisé par le fait que ledit plasma enrichi contient au moins 50 millions de plaquettes par ml.

3. Procédé selon la revendication précédente, caractérisé par le fait que ledit plasma de départ contient de 100 millions à 1 milliard, et en particulier de 100 millions à 500 millions de plaquettes par ml.

4. Procédé selon l'une quelconque des revendications 1 à 3, caractérisé par le fait que l'on réchauffe ledit cryoprécipité pour obtenir ladite colle sous forme liquide.

5. Procédé selon l'une quelconque des revendications 1 à 3, caractérisé par le fait qu'au départ de sang prélevé, on prépare d'abord un plasma enrichi en plaquettes.

6. Procédé selon la revendication précédente, caractérisé par le fait que ledit plasma provient d'un seul donneur, et que l'on effectue l'ensemble des opérations dudit procédé dans un système de poches servant à prélever le sang.

7. Utilisation autre que dans une méthode de traitement thérapeutique ou chirurgical du corps humain ou animal de la colle biologique obtenue selon le procédé de l'une quelconque des revendications précédentes, caractérisée par le fait que l'on ajoute à ladite colle un agent favorisant l'activation de la prothrombine endogène.

8. Utilisation selon la revendication 7, caractérisée par le fait que ledit agent est une solution aqueuse contenant des ions calcium.

9. Utilisation selon la revendication 7, caractérisée par le fait que ledit agent est la thromboplastine, en présence d'ions calcium.

10. Utilisation selon la revendication 7, caractérisée par le fait que ledit agent est un activateur du Facteur XII de la coagulation, en présence d'ions calcium.

11. Utilisation selon la revendication précédente, caractérisé par le fait que ledit activateur est choisi parmi des composés solides insolubles dans la colle biologique et présentant des charges négatives.

12. Utilisation selon la revendication précédente, caractérisée par le fait que ledit composé solide est choisi parmi les silicates, le verre de silice, du carbonate de calcium ou du tri-calcium dicitrate.

## Claims

1. A method of preparing a biological adhesive, in which a cryoprecipitate, constituting said adhesive, is prepared and recovered from a frozen plasma, wherein a platelet-enriched plasma is used as the starting plasma.

2. The method according to claim 1, wherein said enriched-plasma contains at least 50 million platelets per ml.

3. The method according to the preceding claim, wherein said starting plasma contains from 100 million to 1 billion platelets per ml, and in particular from 100 million to 500 million platelets per ml.

4. The method according to any one of claims 1 to 3, wherein said cryoprecipitate is heated in order to obtain said adhesive in liquid form.

5. The method according to any one of claims 1 to 3, wherein a platelet-enriched plasma is prepared in first place from collected blood.

6. The method according to the preceding claim, wherein said plasma is obtained from one donor, and all steps of said method are carried out in a system of bags used for collecting blood.

7. Use other than in a method for treatment of the human or animal body by surgery or therapy ; of a biological adhesive obtained according to the method of any one of the preceding claims, wherein an agent which promotes activation of endogenous prothrombin is added to said adhesive.

8. Use according to claim 7, wherein said agent is an aqueous solution containing calcium ions.

9. Use according to claim 7, wherein said agent is thromboplastin, in the presence of calcium ions.

10. Use according to claim 7, wherein said agent is an activator of coagulation factor XII, in the presence of calcium ions.

11. Use according to the preceding claim, wherein said activator is selected from solid compounds which are insoluble in the biological adhesive and have negative charges.

12. Use according to the preceding claim, wherein said solid compound is selected from silicates, silica glass, calcium carbonate and tricalcium dicitrate.

## Patentansprüche

1. Verfahren zur Herstellung eines biologischen Klebstoffs, in welchem ein den Klebstoff bildendes Cryopräzipitat aus einem eingefrorenen Blutplasma hergestellt und gewonnen wird, **dadurch gekennzeichnet, dass** als Ausgangsblutplasma ein mit Blutplättchen angereichertes Blutplasma eingesetzt wird.

2. Verfahren nach Anspruch 1, **dadurch gekennzeichnet, dass** das angereicherte Blutplasma mindestens 50 Millionen Blutplättchen pro ml enthält.

3. Verfahren nach dem vorhergehenden Anspruch, **dadurch gekennzeichnet, dass** das Ausgangsblutplasma 100 Millionen bis 1 Milliarde und insbesondere 100 Millionen bis 500 Millionen Blutplättchen pro ml enthält.

4. Verfahren nach einem der Ansprüche 1 bis 3, **dadurch gekennzeichnet, dass** das Cryopräzipitat wieder erwärmt wird, um den Klebstoff in flüssiger Form zu erhalten.

5. Verfahren nach einem der Ansprüche 1 bis 3, **dadurch gekennzeichnet, dass,** ausgehend von entnommenem Blut, zunächst ein mit Blutplättchen angereichertes Blutplasma hergestellt wird.

6. Verfahren nach dem vorhergehenden Anspruch, **dadurch gekennzeichnet, dass** das Blutplasma von einem einzigen Spender stammt, und dass alle Stufen des Verfahrens in einem System aus Beuteln, das zur Blutentnahme dient, durchgeführt werden.

7. Verwendung des biologischen Klebstoffs, der gemäß dem Verfahren nach einem der vorhergehenden Ansprüche erhalten worden ist, die anders ist als in einem verfahren zur therapeutischen oder chirurgischen Behandlung eines menschlichen oder tierischen Körpers, **dadurch gekennzeichnet, dass** dem Klebstoff ein Mittel zugesetzt wird, das die Aktivierung von endogenem Prothrombin fördert.

8. Verwendung nach Anspruch 7, **dadurch gekennzeichnet, dass** das Mittel eine Calciumionen enthaltende wässrige Lösung ist.

9. Verwendung nach Anspruch 7, **dadurch gekennzeichnet, dass** das Mittel Thromboplastin in Gegenwart von Calciumionen ist.

10. Verwendung nach Anspruch 7, **dadurch gekennzeichnet, dass** das Mittel ein Aktivator des Blutgerinnungsfaktors XII in Gegenwart von Calciumionen ist.

11. Verwendung nach dem vorhergehenden Anspruch, **dadurch gekennzeichnet, dass** der Aktivator aus festen Verbindungen, die im biologischen Klebstoff unlöslich sind und negative Ladungen tragen, ausgewählt ist.

12. Verwendung nach dem vorhergehenden Anspruch, **dadurch gekennzeichnet, dass** die feste Verbindung aus Silicaten, Siliciumdioxidglas, Calciumcarbonat oder Tricalciumdicitrat ausgewählt ist.
